# EUROPEAN PATENT APPLICATION

(11) **EP 3 838 233 A1**
(43) Date of publication of application: **23.06.2021**
(21) Application number: 19218315.0
(22) Date of filing: 19.12.2019
(51) Int. Cl.: A61F 5/01, A61F 5/058

(54) **ORTHOSIS FOR PLACEMENT ON A HUMAN HAND**

(71) Applicant: Manometric Holding B.V., 2629 JD Delft (NL)
(72) Inventor: Smakman, Pieter, 2629 JD Delft (NL); Dooley, Erin Rae, 2629 JD Delft (NL); Janssen, Daan Iskander, 2629 JD Delft (NL); Halm, Max, 2629 JD Delft (NL)
(74) Representative: De Vries & Metman

(57) **Abstract**

The invention relates to an orthosis (1) for wearing on a human hand (2) to restrict motion of at least the carpometacarpal joint (CMCj) of the thumb and preferably also of the metacarpophalangeal joint (MCPj) of the thumb (2-1), the orthosis (1) comprising a metacarpus section (3), which, when the orthosis (1) is worn on a hand, extends across the metacarpals of digits 2-5, and a thumb section (4), which section is curved or annular in shape and fits around the thumb (2-1), preferably around the proximal phalanx (6) of the thumb (2-1). The thumb section (4) is connected to the metacarpus section (3) via a thumb metacarpal section (7) that, when the orthosis (1) is worn on a hand (2), extends along and/or around the thumb metacarpal (8).

## Description

The invention relates to an orthosis for wearing on a human hand to restrict motion of at least the carpometacarpal joint of the thumb (also referred to as the CMC1 joint) and preferably also of the metacarpophalangeal joint of the thumb (the MCP1 joint), the orthosis comprising a metacarpus section, which, when the orthosis is worn on a hand, extends across the metacarpals of digits 2-5, and a thumb section, which section is curved or annular in shape and fits around the thumb, preferably around the proximal phalanx of the thumb.

US 2019/053931 relates to an orthosis for fastening to a human hand for immobilizing the carpometacarpal joint and metacarpophalangeal joint of the thumb of the hand, which has a stiff body which can be of multi-piece or single-piece design. The rigidity of the body enables the latter to support, to stabilize and to immobilize the hand and dimensional stability is provided such that the body can be placed in a simple manner with a defined shape onto the hand.

The WE CMC1/MP1 stabilisation brace, also known as silver splint, is made from sterling silver and holds CMC1 and MCP1 in a fixed position. The brace is applied in case of arthritis. It has a metacarpus section and a thumb ring, which ring is soldered directly to the metacarpus section.

Certain pathologies and injuries require immobilization of the carpometacarpal (CMC) joint and the metacarpophalangeal (MCP) joint. An example of a pathology is arthritis, which in its two most common forms, to wit degenerative arthritis (osteoarthritis) and rheumatoid arthritis, affects the cartilage and capsula of these joints. The degeneration of the joint causes pain and stiffness. In the long term, the joint deforms, affecting corresponding muscles and bones. This results in a less functional hand that limits people during their everyday lives.

Examples of injuries of the CMC1 and/or MCP1 joints are repetitive strain injury (RSI) and skiers thumb. RSI is caused by daily repetitive movements, overexerting certain muscles and tendons, which causes irritation and inflammation to these muscles and tendons. Skier's thumb is a strained or even torn ulnar collateral ligament (UCL) that is caused e.g. by falling onto a ski pole, extending the MCP1 joint too far. Although its name suggests otherwise, skier's thumb results frequently from other sports, such as volleyball or basketball. To heal a sprained or partly torn tendon, stabilization of the thumb is crucial.

It is an object of the present invention to provide an improved orthosis.

To this end, the orthosis according to the present invention is characterised in that the thumb section is connected to the metacarpus section via a thumb metacarpal section that, when the orthosis is worn on a hand, extends along and/or around the thumb metacarpal.

The thumb metacarpal section according to the present invention provides supported motion of the thumb, which sufficiently restricts motion to prevent pain or damage and which, at the same time, provides support for the thumbcarpals. Further, in use, the wearer can still move his/her thumb by applying force in a certain direction and elastically deforming the orthosis. When the wearer stops applying force, resilience causes the splint to return to its rest position and, in doing so, guides the thumb to its natural position. The forces generated by the movements of the thumb are transmitted via the thumb metacarpal section to the metacarpus section and distributed over the palmar and/or dorsal side of the hand.

Although the orthosis can be articulated, e.g. by connecting the thumb section to the thumb metacarpal section and/or connecting the thumb metacarpal section to the metacarpus section by a flexible or rotary and e.g. spring-loaded joint or joints, it is preferred that the thumb metacarpal section is resilient. Resilience can be achieved e.g. through the choice of material, the thickness of the material and/or through construction.

In an embodiment, the thumb metacarpal section is cantilevered to the metacarpus section. It is preferred that it is connected to the metacarpus section at its proximal end or base and that there are no other connections, such as solders, welds or bridges, between the two. In another embodiment, the thumb metacarpal section extends helically between the metacarpus section and the thumb section, preferably over at least 180°, i.e. at least a half-turn. These embodiments provide effective ways of achieving, on the one hand, a degree of freedom of movement while, on the other hand, maintaining sufficient support and providing resilience to return the splint to its rest position.

In an embodiment, the reaction force on the thumb section is substantially proportional to the movement of the thumb. The relation between reaction force and movement of the thumb depends on several aspects and is thus complex and difficult to quantify precisely. In an embodiment, the relation between the reaction force on the thumb section and the movement of the thumb is linear ± 40% or linear ± 20%. I.e., although not itself linear the relation, typically a curve, extends within ± 40% of a linear relation.

In an embodiment, motion of the thumb is restricted to a percentage in a range from 5% to 95%, preferably from 15% to 85%, preferably from 30% to 70%, preferably from 40% to 60%, of the wearer's total range of motion, in at least one of the directions of movement referred to as adduction, abduction, flexion, and extension.

In an example, a higher stiffness would be selected for patients that need more support, e.g. if more restriction of motion is needed and/or if the user has more than average strength in his or her hand. A lower stiffness would be selected for users that need less support, e.g. in case of less severe arthritis and/or the user has less than average strength in his or her hand. By achieving or more accurately approaching the minimum support necessary for the treatment, compliance typically increases because wearing the orthosis is less restrictive and more comfortable for the patient. A higher compliance in turn increases the efficiency of the treatment and helps prevent the necessity of surgery.

In an embodiment, when the orthosis is worn on a hand, the thumb metacarpal section extends over (across) the palmar side of the metacarpophalangeal (MCP) joint.

In another embodiment, the connection or transition between the thumb metacarpal section and the metacarpus section is located on the dorsal side of the hand, preferably proximal to, i.e. beneath, the MCP joint of the index finger and following a line halfway between the thumb CMC and MCP joints to wrap around to the palmar side of the hand at the MCP joint.

In another embodiment, the metacarpus section, when the orthosis is worn on a hand, extends on the palmar side of the hand. In a refinement, the metacarpus section extends proximal to the MCP joints of digits 2-5.

In an embodiment, the thumb section comprises an open loop which, when the orthosis is worn on a hand, surrounds the thumb, preferably halfway between the MCP and inter phalangeal (IP) thumb joints. The small opening allows the thumb loop circumference to flex, facilitating placing and removing the orthosis.

In an embodiment, to provide e.g. smooth curves and/or gradients in e.g. support, stiffness, and/or resilience along the length of the orthosis, the orthosis is made from a single piece, such as a plastically deformed plate, strip or bar, or a continuous element, e.g. a molded or 3D printed element.

For instance, in an embodiment, the width and/or the thickness of the orthosis varies along its length. The width will normally be at a maximum at both the ulnar and radial tabs as this provides the most comfortable distribution of pressure. The upper thumb loop is the narrowest section as this area of the hand is the smallest and to avoid interfering with IP joint flexion and extension or with performing everyday tasks.

In an embodiment, the widths are in a range from 3 to 50 mm, preferably in a range from 4 to 20 mm and/or the thicknesses are in a range from 1 to 15 mm, preferably in a range from 2 to 12 mm.

As mentioned above, resilience, and thus the dependence of the reaction force on the thumb section of deflection of the thumb, can be achieved e.g. through the choice of material, the thickness of the material and/or through the (shape of the) construction. It will typically be different for each direction (anisotropy). Suitable materials include thermoplastic materials, e.g. polymers, such as cellulose acetate, polylactic acid (PLA), and polyamides (Nylons), and metals, in particular metals having a high yield strength, such as titanium and Monel.

For the sake of completeness, attention is drawn to the following prior art.

WO 2014/070621 (EP 2 914 218) relates to a spiral brace that "has an inner surface that corresponds to a digital representation of an injured limb. The spiral brace can wrap around a length of the limb in a helical manner. The body of the spiral brace may be a single piece structure that is fenestrated to provide air circulation to the injured limb. The invention will now be explained in more detail with reference to the drawings, which show a preferred embodiment of the present invention." Figures 12 to 15 illustrate a spiral brace that restricts thumb movement.

US 4,854,310 relates to a splint to immobilize part of a limb having an elongated rigid sheet, a rigid collar and at least one retaining strap. The embodiment shown in Figures and 1 and 2 "the splint 10 also has a rigid collar 17 in the shape of an elongated letter "C" extending circumferentially more than halfway around the hand 18.... Optionally, a stem 19, connected to collar 17, extends in the direction of the thumb 14 to a ring 110. The ring 110 is made of a strip of stainless steel and it is connected to stem 19. Ring 110 is bent into almost a full circle to fit around the thumb. The combination of stem 19 and ring 110 prevents lateral motion of the thumb 14 relative to the collar 17."

Within the framework of the present invention the words "connected to" and "connection" in relation to the thumb, metacarpus section, and thumb metacarpal sections include continuous transitions, e.g. when the orthosis is made from a single piece of materials. The word "orthosis" includes braces and splints.
Figures 1 to 3 are perspective views of a first embodiment of the orthosis according to the present invention, worn on a hand and with the metacarpus section extending on the palmar side of the hand.
Figures 4 to 7 are perspective views of the first embodiment of the orthosis, removed from the hand.
Figure 8 is a graph illustrating different degrees of resilience.
Figure 9 is a perspective view of a second embodiment of the orthosis according to the present invention, worn on a hand and with the metacarpus section extending on the dorsal side of the hand.

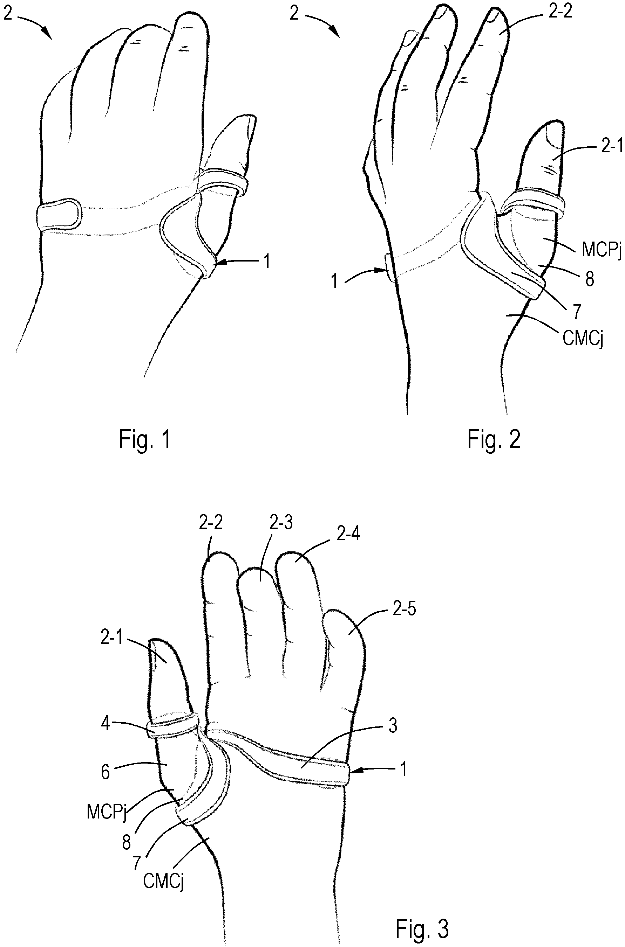

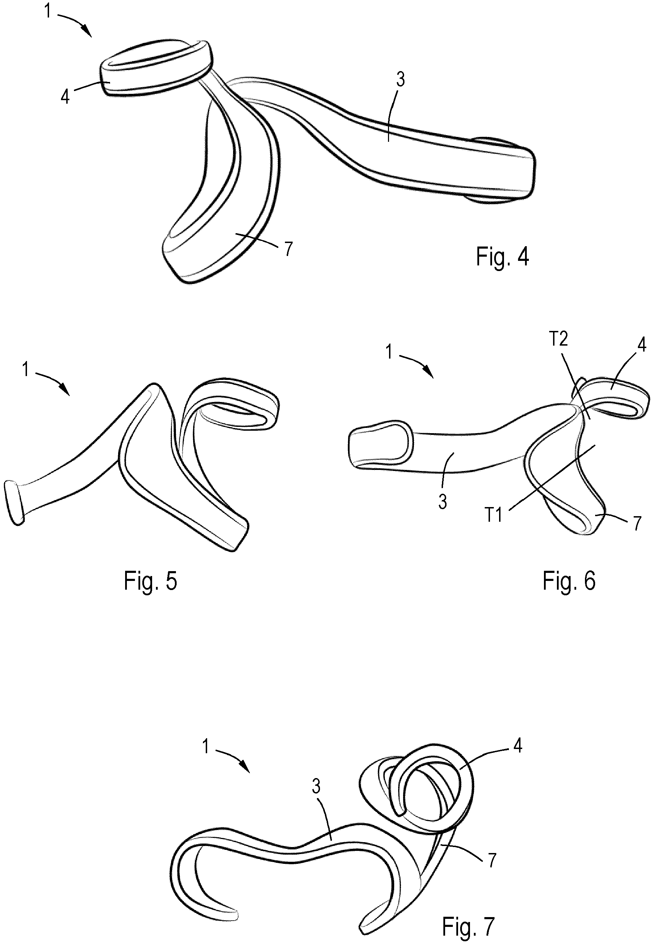

Figures 1 to 3 show a first orthosis 1 according to the present invention, worn on a hand 2 to restrict motion of the carpometacarpal joint, CMCj, and the metacarpophalangeal joint, MCPj, of the thumb 2-1. Figures 4 to 7 show the same orthosis removed from the hand 2. The orthosis 1 comprises a metacarpus section 3, which, when the orthosis is worn on a hand, extends across the metacarpals of digits 2-5, i.e., the index finger (digit 2; reference sign 2-2 in the Figures), middle finger (digit 3; 2-3), ring finger (digit 4; 2-4) and little finger (digit digit 5; 2-5), and a thumb section 4. The thumb section 4 is curved in shape and fits around the proximal phalanx 6 of the thumb 2-1.

The orthosis is a single piece of a thermoplastic material, such as cellulose acetate or polyamide, and made by molding or additive manufacturing followed by polishing.

The thumb section 4 is connected to the metacarpus section 3 via a thumb metacarpal section 7 that, when the orthosis 1 is worn on a hand 2, extends along or around the thumb metacarpal (MCP1) 8.

In this example, the metacarpus section 3 extends on the palmar side of the hand, follows the shape of the palm of the hand, and 'hooks' around the ulnar and radial sides of the hand. The metacarpus section is open on one side, in this example on the dorsal side of the hand, to facilitate placing it on the hand. Once in place, the metacarpus section provides the basis for supporting the resilient thumb metacarpal section and the thumb section.

The thumb section 4 consists of an open loop which, when the orthosis is worn on a hand, surrounds the thumb, between the MCP and IP thumb joints. The small opening allows the thumb loop circumference to flex, facilitating placing and removing of the orthosis.

As best shown in Figures 1 and 6, the thumb metacarpal section 7 is cantilevered to the metacarpus section 3 and extends helically between the metacarpus section 3 and the thumb section 4, over almost a full turn. In this example, the thumb metacarpal section 7 transitions to the metacarpus section 3 at its proximal end and there are no other connections between the two sections 7, 3. As the orthosis was made in a single piece, the connection is not discrete but instead a continuous transition between the two sections. The transition is roughly indicated by means of a line, T1, in Figure 6. The same is true for the transition between the thumb section 4 and the thumb metacarpal section 7, which is indicated, in Figure 6, by a second line, T2.

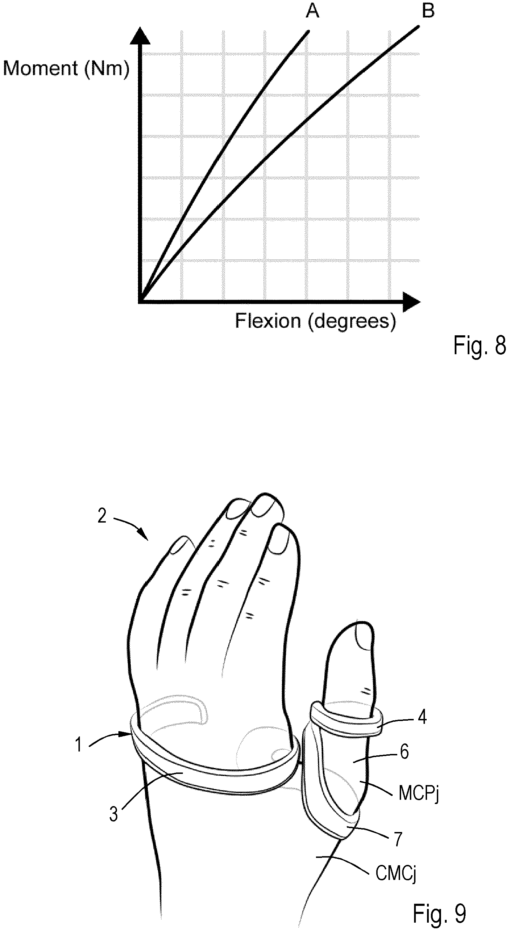

Figure 8 is a graph that schematically illustrates the reaction force on the thumb section dependent on flexion on the thumb, wherein curve A represents a material and/or construction that has a relatively high stiffness in the direction of flexion, whereas curve B represents a material and/or construction that has a relatively low stiffness in the direction of flexion.

The orthosis according to the present invention provides supported motion of the thumb, while reducing the risk of pain or damage. The wearer can still move his/her thumb by applying force in a certain direction and elastically deforming the orthosis. When the wearer stops applying force, resilience causes the splint to return to its rest position and, in doing so, guides the thumb to its natural position. The orthosis can be compact, not bulky, unobtrusive, and when a material having a low thermal conductivity is used, feels warm. The construction in the example above requires no closure.

In an example, a patient has degenerative arthritis degree 2 (Kellgren-Lawrence classification) in the CMC1 and MCP1 joints of his right hand. The wrist and other finger joints have no arthritis. The patient is a male, 36 years old, and works as a farmer, which implies that most of his work is intensive manual labor. There is no limitation in the range of motion (ROM), he is able to exert a pinch force, with his right thumb of 12 kilograms, and during manual labour pain, measured in accordance with the Numerical Rating Scale (NRS), is up to 8/10. To properly support this man's thumb, the orthosis needs to limit both the CMC1 and MCP1 joints. The total ROM needs to be reduced by 80% at a force of 7,5 kg. This ROM limitation means that the total allowed flexion ROM with the orthosis in the CMC1 joint is 1 cm when applying a force of 7,5 kg.

The invention is not restricted to the above-described embodiments, which can be varied in a number of ways within the scope of the claims. E.g., instead of open on the dorsal side of the hand, the metacarpus section could be closed or open on the palmar side of the hand, as illustrated in Figure 9.

## Claims

1. Orthosis (1) for wearing on a human hand (2) to restrict motion of at least the carpometacarpal joint (CMCj) of the thumb and preferably also of the metacarpophalangeal joint (MCPj) of the thumb (2-1), the orthosis (1) comprising a metacarpus section (3), which, when the orthosis (1) is worn on a hand, extends across the metacarpals of digits 2-5, and a thumb section (4), which section is curved or annular in shape and fits around the thumb (2-1), preferably around the proximal phalanx (6) of the thumb (2-1), **characterised in that** the thumb section (4) is connected to the metacarpus section (3) via a thumb metacarpal section (7) that, when the orthosis (1) is worn on a hand (2), extends along and/or around the thumb metacarpal (8).

2. Orthosis (1) according to claim 1, wherein the thumb metacarpal section (7) is cantilevered to the metacarpus section (3).

3. Orthosis according to claim 1 or 2, wherein the thumb metacarpal section (7) extends helically between the metacarpus section (3) and the thumb section (4), preferably over at least 180°.

4. Orthosis (1) according to any one of the preceding claims, wherein the reaction force on the thumb section (4) is proportional to the movement of the thumb (2-1) .

5. Orthosis (1) according to any one of the preceding claims, wherein motion of the thumb (2-1) is restricted to a percentage in a range from 5% to 95%, preferably from 15% to 85%, preferably from 30% to 70%, preferably from 40% to 60%, of the wearers total range of motion.

6. Orthosis (1) according to any one of the preceding claims, wherein, when the orthosis (1) is worn on a hand (2), the thumb metacarpal section (7) extends over the palmar side of the metacarpophalangeal joint (MCPj).

7. Orthosis (1) according to any one of the preceding claims, wherein the connection between the thumb metacarpal section (7) and the metacarpus section (3) is located on the dorsal side of the hand (2).

8. Orthosis (1) according to any one of the preceding claims, wherein the thumb section (4) comprises an open loop.

9. Orthosis (1) according to any one of the preceding claims, wherein the metacarpus section (3), when the orthosis (1) is worn on a hand, extends on the palmar side of the hand (2).

10. Orthosis (1) according to claim 9, wherein the metacarpus section (3) extends proximal to the MCP joints of digits 2 to 5 (2-2 ... 2-5) .

11. Orthosis (1) according to any one of the preceding claims, wherein the orthosis (1) is made from a single piece, such as a plastically deformed plate, strip or bar, or a continuous, e.g. molded or 3D printed, element.

12. Orthosis (1) according to any one of the preceding claims, wherein the width and/or the thickness of the orthosis (1) varies along its length.

13. Orthosis (1) according to claim 12, wherein the widths are in a range from 3 to 50 mm, preferably in a range from 4 to 20 mm.

14. Orthosis (1) according to claim 12 or 13, wherein the thicknesses are in a range from 1 to 15 mm, preferably in a range from 2 to 12 mm.
